(19)

Europäisches
Patentamt
European
Patent Office
Office européen
des brevets

(11) **EP 3 466 424 A1**

(12) **EUROPEAN PATENT APPLICATION**
published in accordance with Art. 153(4) EPC

(43) Date of publication:
**10.04.2019 Bulletin 2019/15**

(21) Application number: **17802801.5**

(22) Date of filing: **23.05.2017**

(51) Int Cl.:
*A61K 31/436* [(2006.01)]   *A61K 47/02* [(2006.01)]
*A61K 47/26* [(2006.01)]   *A61K 47/36* [(2006.01)]
*A61P 31/04* [(2006.01)]   *A61P 35/00* [(2006.01)]

(86) International application number:
**PCT/JP2017/019192**

(87) International publication number:
**WO 2017/204215 (30.11.2017 Gazette 2017/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.05.2016 JP 2016105735**

(71) Applicant: **Nippon Kayaku Kabushiki Kaisha Tokyo 100-0005 (JP)**

(72) Inventor: **KAWAMURA Dai Tokyo 115-8588 (JP)**

(74) Representative: **Godemeyer Blum Lenze Patentanwälte Partnerschaft mbB - werkpatent An den Gärten 7 51491 Overath (DE)**

(54) **PHARMACEUTICAL COMPOSITION COMPRIZING RAPAMYCIN OR DERIVATIVE THEREOF**

(57)    It is an object of the present invention to provide a solid pharmaceutical composition, which can suppress a reduction in the content of an active ingredient caused by decomposition of rapamycin or a derivative thereof due to light irradiation, can ensure long-term stability, and has high storage convenience. In the present invention, the inventors have found that a preparation comprising rapamycin or a derivative thereof wherein a iron oxide is dispersed in the preparation can ensure light stability of the active ingredient, thereby completing the present invention. The present invention relates to a solid pharmaceutical preparation comprising rapamycin or a derivative thereof and iron oxide, wherein the iron oxide is dispersed in the solid pharmaceutical preparation.

**EP 3 466 424 A1**

**Description**

Technical Field

The present invention relates to a pharmaceutical preparation composition, in which the stability of rapamycin or a derivative thereof is improved. The rapamycin or a derivative thereof is extremely unstable against light, oxygen and water. In particular, the rapamycin or a derivative thereof has extremely low stability under light irradiation, and thus, is problematic in terms of storage stability. The present invention relates to a technique of improving the storage stability of such a compound under light irradiation, thereby providing a pharmaceutical composition having high storage convenience.

Background Art

It has been known that rapamycin (sirolimus) is a macrolide antibiotic discovered from the metabolite of actinomyces and has an immunosuppressive action. Rapamycin has an action to inhibit a mammalian rapamycin target protein (mammalian target of rapamycin; mTOR) that regulates cell division, cell growth, survival, etc. This mTOR is a main serine-threonine kinase, which regulates the synthesis of proteins by stimulation with growth factors, nutrients, etc., and the mTOR has been known to regulate the growth, proliferation and survival of cells, and angiogenesis. Hence, the mTOR inhibitory action of rapamycin has been focused, and the synthesis of a derivative thereof has been attempted. As a result, everolimus and temsirolimus have been discovered as antitumor agents.

A pharmaceutical formulation used to provide a pharmaceutical product comprising rapamycin or a derivative thereof has been reported. Patent Literature 1 discloses that a solution containing a mixture of rapamycin, hydroxypropylmethyl cellulose, lactose and the like is prepared, the solvent is then distilled away from the solution, and the obtained solid dispersion is then formulated. In addition, Patent Literature 2 discloses a tablet comprising everolimus used as a rapamycin, crospovidone used as a disintegrator, colloidal silicon dioxide, and lactose.

Rapamycin or a derivative thereof has been known to have physical properties by which it is extremely instable to light, oxidation and moisture. Hence, an antioxidant is added to a pharmaceutical product formulation comprising, as an active ingredient, rapamycin. For instance, to a rapamycin preparation (registered trademark: Rapalimus) and a temsirolimus preparation (registered trademark: Torisel), tocopherol is added. In addition, for everolimus formulations (registered trademark: Afinitor and Certican), a synthetic antioxidant, dibutylhydroxytoluene (BHT) is used.

Regarding a method of efficiently stabilizing a rapamycin derivative using an antioxidant, Patent Literature 3 discloses that a mixed solution containing everolimus and BHT as an antioxidant is prepared, and the solvent is then removed from the mixed solution, so as to obtain a stabilized everolimus solid.

Moreover, Patent Literature 4 reports that an ethanol solution of everolimus is added to a water-soluble polymer such as hypromellose, and the mixture is then granulated to prepare a solid dispersion, thereby obtaining a stable everolimus composition without using antioxidants.

However, none of the aforementioned techniques regarding a preparation comprising a stable rapamycin derivative relate to a method of stabilizing the rapamycin or a derivative thereof against light. Accordingly, currently commercially available everolimus preparations (Afinitor (registered trademark) tablets and Certican (registered trademark) tablets) are both distributed on the market in the form of a press-through package (PTP package), both surfaces of which are made of aluminum, which is different from a common tablet package form.

As a common method of stabilizing a pharmaceutical product tablet against light, a method of preparing a film-coated tablet, using a coating film containing a light-shielding agent, has been known. For example, Patent Literature 5 reports that the safety of aranidipine unstable against light can be improved by using a coating film containing titanium oxide as a light-shielding agent.

However, since an aqueous solution of a coating agent needs to be used in order to prepare such a film-coated tablet, the aforementioned method cannot be applied to drugs having low chemical stability against aqueous solvents. In particular, it has been found that rapamycin or a derivative thereof causes a rapid reduction in purity in protic solvents such as water or ethanol. For example, everolimus as a rapamycin derivative has physical properties, by which when it is dissolved in ethanol to prepare an ethanol solution thereof, its purity is decreased by 4% or more under conditions of 3 hours/25°C. As such, it is not preferable that a film coating technique involving the use of an aqueous solvent be applied, as a pharmaceutical formulation of light-resistant preparations, to a pharmaceutical product tablet comprising, as an active ingredient, rapamycin and a derivative thereof.

Prior Art Literatures

Patent Literatures

Patent Literature 1: JP Patent Publication (Kohyo) No. 11-509223 A (1999)
Patent Literature 2: JP Patent Publication (Kohyo) No. 2005-507897 A
Patent Literature 3: JP Patent Publication (Kohyo) No. 2002-531527 A
Patent Literature 4: International Publication WO2013/022201
Patent Literature 5: JP Patent Publication (Kokai) No. 2003-104887 A

Summary of Invention

Object to be Solved by the Invention

It is an object of the present invention to provide a solid pharmaceutical preparation comprising, as an active ingredient, rapamycin or a derivative thereof, wherein the solid pharmaceutical preparation is improved in terms of light stability, and does not essentially need to be controlled under strict light-shielding conditions involving hermetical sealing with an aluminum-made packaging material, etc., thereby being excellent in terms of storage convenience.

Means for Solving the Object

The present inventors have found that, in order to obtain a solid pharmaceutical preparation comprising, as an active ingredient, rapamycin or a derivative thereof, which is excellent in terms of light resistance, iron oxide is dispersed in the solid pharmaceutical preparation, so that the solid pharmaceutical preparation can persistently exhibit stabilizing effects under light irradiation, thereby completing the present invention. Specifically, the present application includes the inventions [1] to [7] as a gist thereof.
[1] A solid pharmaceutical preparation comprising rapamycin or a derivative thereof and iron oxide, wherein the iron oxide is dispersed in the solid pharmaceutical preparation.
In the present invention, a composition, in which the stability of rapamycin or a derivative thereof under light irradiation is improved, can be prepared by dispersing iron oxide in a solid preparation comprising the rapamycin or a derivative thereof. In the case of a solid preparation comprising rapamycin or a derivative thereof, when titanium oxide generally known as a light-shielding substance was dispersed in the solid preparation, it did not exhibit stabilizing effects under light irradiation, but it rather promoted decomposition of the rapamycin or a derivative thereof. In order to improve the stability of rapamycin or a derivative thereof under light irradiation, addition of iron oxide is extremely important.
[2] The solid pharmaceutical preparation according to the above [1], wherein the iron oxide is ferric oxide and/or yellow ferric oxide.
The iron oxide is preferably iron oxide used as a pharmaceutical product additive or a food product additive.
[3] The solid pharmaceutical preparation according to the above [1] or [2], wherein the content percentage of the iron oxide in the solid pharmaceutical preparation is 0.01% by mass or more and 2% by mass or less.
[4] The solid pharmaceutical preparation according to any one of the above [1] to [3], which comprises sugars having a critical relative humidity at 25°C of 95% or more.
[5] The solid pharmaceutical preparation according to any one of the above [1] to [4], wherein the rapamycin or a derivative thereof is dispersed in the solid pharmaceutical preparation in the form of a solid dispersion comprising an antioxidant and sugars having a critical relative humidity at 25°C of 95% or more, and the iron oxide is dispersed in the solid pharmaceutical preparation in the form of a mixture with the solid dispersion.
The solid pharmaceutical preparation of the present invention is prepared by mixing a solid dispersion comprising rapamycin or a derivative thereof with iron oxide. The solid dispersion is a granular composition comprising an active ingredient and any given pharmaceutical additives, and in a preferred embodiment, the solid dispersion and the iron oxide are dispersed in the solid pharmaceutical preparation.
[6] The solid pharmaceutical preparation according to the above [5], wherein the solid dispersion comprising the rapamycin or a derivative thereof comprises a water-soluble cellulose derivative.
In a preferred embodiment, the solid dispersion is a granular composition comprising the rapamycin or a derivative thereof and the water-soluble cellulose derivative.

[7] The solid pharmaceutical preparation according to the above [5] or [6], wherein the solid dispersion comprising the rapamycin or a derivative thereof, the iron oxide, and a lubricant are dispersed in the solid pharmaceutical preparation.

Advantageous Effects of Invention

Rapamycin or a derivative thereof has physical properties by which it has poor light stability and the content of the active ingredient is decreased under light irradiation. According to the solid pharmaceutical preparation of the present invention, a solid pharmaceutical preparation comprising rapamycin or a derivative thereof, which is excellent in term of light stability and does not essentially need to be controlled under strict light-shielding conditions, thereby being improved in terms of storage convenience, can be provided.

Embodiments for Carrying out the Invention

The solid pharmaceutical preparation of the present invention is characterized in that it is a pharmaceutical preparation comprising rapamycin or a derivative thereof and iron oxide, and in that the iron oxide is dispersed in the solid pharmaceutical preparation. That is to say, in general, when iron oxide is applied to a pharmaceutical preparation, a preparation, in which iron oxide is dispersed in a coating film and is then localized in the surface layer of a tablet, has been known. In contrast, the present invention is characterized in that such iron oxide is dispersed in a solid pharmaceutical preparation. The details thereof will be described below.

The present invention comprises, as an active ingredient, rapamycin or a derivative thereof.

Rapamycin (common name: Sirolimus) is a compound having a macrolide skeleton that has been isolated from the metabolite of actinomyces, *Streptomyces Hygroscopicus*, separated from the soil of Easter Island.

The term "rapamycin derivative" means a substance prepared by chemically modifying rapamycin used as a mother core. Examples of the rapamycin derivative include 16-O-substituted rapamycin (see, for example, WO 94/022136), 40-O-substituted rapamycin (see, for example, US 5258389 and WO 94/09010), carboxylic acid ester-substituted rapamycin (see, for example, WO 92/05179), amide-substituted rapamycin (see, for example, US 5118677), fluorine-substituted rapamycin (see, for example, US 5100883), and acetal-substituted rapamycin (see, for example, US 5151413). The rapamycin or a derivative thereof of the present invention is not limited thereto, but the aforementioned rapamycin derivatives can be used as applicable preferred compounds.

The rapamycin derivative is preferably a 40-O-substituted rapamycin derivative, in which the hydroxyl group at position 40 of the cyclohexyl group of rapamycin is substituted with a hydroxyalkyl group, a hydroxyalkoxyalkyl group, an acylaminoalkyl group, an aminoalkyl group, or a hydroxy-substituted acyl group. The rapamycin derivative is more preferably 40-O-(2-hydroxyethyl)rapamycin (everolimus), or 40-O-[3-hydroxy-2-(hydroxymethyl)-2-methylpropanoate]rapamycin (temsirolimus).

As (A) rapamycin or a derivative thereof of the present invention, rapamycin (Sirolimus), everolimus, or temsirolimus is preferably used.

As such (A) rapamycin or a derivative thereof, a compound having a quality that can be sufficiently used as a pharmaceutical product is preferably used.

The iron oxide used in the present invention is not particularly limited, as long as it is a substance in which iron is oxidized. Examples of the iron oxide include ferrous oxide, triiron tetraoxide, ferric oxide, yellow ferric oxide, and yellow iron oxide. The iron oxide is preferably ferric oxide or yellow ferric oxide. As such iron oxide, iron oxide, which has been approved as an additive for pharmaceutical products and/or an additive for food products, is preferably used.

In the present invention, as iron oxide, the above-described iron oxides may be used each alone, or may also be used in combination of two or more types.

With regard to the amount of iron oxide added into the solid pharmaceutical preparation of the present invention, the iron oxide is preferably used at a content percentage of 0.01% by mass or more in the solid pharmaceutical preparation. The iron oxide is used at a content percentage of more preferably 0.1% by mass or more, and further preferably 0.5% by mass or more. Since iron oxide is not particularly problematic in terms of safety, the upper limit of the use amount thereof is not particularly limited. The upper limit of the use amount should be determined within an amount range that is practically usable as a pharmaceutical product, and it is suitably approximately 5.0% by mass. The upper limit of the use amount of the iron oxide is preferably 2.0% by mass.

Taking into consideration the ensuring of the stability of rapamycin or a derivative thereof and the realistic use amount of pharmaceutical product additives, the iron oxide is preferably used at a content percentage of 0.01% by mass or more and 5% by mass or less in the solid pharmaceutical preparation. The use amount of the iron oxide is more preferably 0.01% by mass or more and 2.0% by mass or less, further preferably 0.1% by mass or more and 2.0% by mass or less, and still further preferably 0.5% by mass or more and 2.0% by mass or less.

The iron oxide is preferably used in an amount of 0.05 to 5 parts by mass with respect to 1 part by mass of the rapamycin or a derivative thereof. The iron oxide is more preferably used in an amount of 0.1 to 1 part by mass.

The solid pharmaceutical preparation of the present invention is a preparation for use in oral administration. Examples of the solid pharmaceutical preparation include a powdered medicine, a granule, a capsule, a troche, a tablet, and a pill. Examples of the tablet include an orally disintegrating tablet, a chewable tablet, an effervescent tablet, a dispersible tablet, and a dissolving tablet. The solid pharmaceutical preparation of the present invention is preferably applied as a tablet. That is to say, the solid pharmaceutical preparation of the present invention is preferably a tablet comprising, as an active ingredient, rapamycin or a derivative thereof, which is prepared as a pharmaceutical composition by making a tablet from the rapamycin or a derivative thereof, the iron oxide, and suitable pharmaceutical additives.

In the solid pharmaceutical preparation of the present invention, the state in which the iron oxide is dispersed in the solid pharmaceutical preparation indicates a state in which the iron oxide is present not only in the surface layer of the preparation but at least a portion thereof is present also in the inner layer of the solid pharmaceutical preparation, and also, the iron oxide is not localized as an aggregate.

As a method of dispersing the iron oxide in the solid preparation, there is applied, for example, a method which comprises mixing rapamycin or a derivative thereof with iron oxide and preparation additives approved as pharmaceutical product preparations, then granulating the mixture, then further adding any given additives for pharmaceutical preparations to the resultant, and then molding the mixture into a tablet. The iron oxide may have previously been added and mixed, upon preparation of a granulated substance containing the rapamycin or a derivative thereof. Otherwise, a granulated substance comprising the active ingredient that does not contain the above-described iron oxide may be first prepared, and thereafter, iron oxide, together with any given pharmaceutical additives, may be mixed with the granulated substance. Alternatively, a granulated substance comprising the active ingredient that contains the above-described iron oxide may be first prepared, and iron oxide, together with any given pharmaceutical additives, may be further mixed with the granulated substance. By molding such a mixture for use in preparing formulations into a tablet, a state in which the iron oxide is dispersed in the solid pharmaceutical preparation can be created.

In the present invention, the method of granulating the rapamycin or a derivative thereof, together with iron oxide and/or pharmaceutical additives approved as compositions, is not particularly limited, as long as it is a method used in the present technical field. Examples of such a granulation method include wet granulation (extrusion granulation, agitation granulation, fluidized bed granulation, spray drying granulation, etc.) and dry granulation.

In the present invention, sugars having a critical relative humidity at 25°C of 95% or more are preferably used in the above-described solid pharmaceutical preparation. The phrase "a critical relative humidity at 25°C of 95% or more" is used to mean that the sugars have physical properties by which the sugars hardly absorb moisture, when the relative humidity at 25°C is 95% or less. In the present invention, all types of sugars can be applied to the solid pharmaceutical preparation without any particular limitation, as long as the sugars have a critical relative humidity at 25°C of 95% or more. Examples of the sugars having such physical properties may include monosaccharides and oligosaccharides, which are generally recognized as sugars having low moisture absorption properties by a person skilled in the art.

As such sugars having a critical relative humidity at 25°C of 95% or more, sugar alcohols or disaccharides are preferable, and further, one or more types of sugars selected from the group consisting of mannitol, lactose, trehalose, and maltose are preferable. These sugars may be used each alone, or may also be used in combination of two or more types. Among others, lactose is particularly preferably used.

In the solid pharmaceutical preparation of the present invention, in the case of using sugars having a critical relative humidity at 25°C of 95% or more, the sugar may be preferably added, at a content percentage of 0.1% by mass or more, into the solid pharmaceutical preparation. Preferably, the sugars may be used at a content percentage of 1% by mass or more. Since sugars do not particularly cause safety hazard, the upper limit of the use amount thereof is not particularly limited. The use amount of the sugars should be determined within an amount range that is practically usable as a pharmaceutical product, and it is suitably approximately 95% by mass. The use amount of the sugars is preferably 90% by mass.

Taking into consideration determination of the suitable content of the rapamycin or a derivative thereof used as an active ingredient and the practically usable amount of pharmaceutical product additives, the iron oxide is preferably used in the solid pharmaceutical preparation at a content percentage of 1% by mass or more and 95% by mass or less. More preferably, the content percentage of the iron oxide is 1% by mass or more and 90% by mass or less.

In a more preferred embodiment of the present invention, an antioxidant is used. The type of such an antioxidant is not particularly limited, as long as it suppresses oxidation of the rapamycin or a derivative thereof and maintains the stability of the active ingredient. As such an antioxidant, a known antioxidant exhibiting the effect of stabilizing the rapamycin and a derivative thereof can be used.

Examples of such a known antioxidant include nitrous acid, ascorbic acid, sodium ascorbate, ascorbyl stearate, ascorbyl palmitate, sulfurous acid, alpha-thioglycerol, edetic acid, erythorbic acid, cysteine hydrochloride, citric acid, sodium citrate, disodium hydrogen citrate, sodium dihydrogen citrate, dichloroisocyanuric acid, dibutylhydroxytoluene (BHT), thioglycolic acid, thiomalic acid, tocopherol, pyrosulfurous acid, butylhydroxyanisole, 1,3-butylene glycol, pentaerythritol tetrakis[3-(3,5-di-tert-butyl-4-hydroxyphenyl)propionate]benzotriazole, isopropyl gallate, 2-mercaptobenzimidazole, and lecithin. These antioxidants may be used each alone, or may also be used in combination of two or more types.

Examples of an antioxidant that is preferably applied herein include sodium ascorbate, ascorbyl stearate, disodium hydrogen citrate, sodium dihydrogen citrate, dibutylhydroxytoluene (BHT), tocopherol, butylhydroxyanisole, isopropyl gallate, and lecithin. Examples of a more preferred antioxidant include sodium ascorbate, ascorbyl stearate, disodium hydrogen citrate, dibutylhydroxytoluene (BHT), tocopherol, and lecithin.

The antioxidant can be used, as appropriate, in an amount that does not impair the stability of the rapamycin or a derivative thereof. The antioxidant is preferably used in an amount of 0.0001 to 10 parts by mass based on 1 part by mass of the rapamycin or a derivative thereof. The amount is more preferably 0.0005 to 1.0 parts by mass and further preferably 0.001 to 1.0 parts by mass.

In a more preferred embodiment of the present invention, a water-soluble polymeric carrier is preferably used. The type of such a water-soluble polymeric carrier is not particularly limited and any type of water-soluble polymeric carrier can be applied, as long as it is a water-soluble polymeric carrier that is applicable as an additive for pharmaceutical products.

Examples of the water-soluble polymeric carrier include cellulosic carriers and synthetic polymeric carriers. Examples of such cellulosic carriers include hydroxypropylmethyl cellulose, hydroxypropyl cellulose, methyl cellulose, ethyl cellulose, carmellose, carmellose sodium, cellacefate, and hypromellose phthalic ester. Examples of such synthetic polymeric carriers include povidone, macrogol, polyvinyl alcohol, and a methacrylic acid copolymer. The above-described water-soluble polymeric carrier may be used each alone, or may also be used in combination.

In the solid pharmaceutical preparation of the present invention, in the case of using the above-described water-soluble polymeric carrier, the water-soluble polymeric carrier is preferably added, at a content percentage of 1% by mass or more, into the solid pharmaceutical preparation. Preferably, the water-soluble polymeric carrier may be used at a content percentage of 10% by mass or more. If the water-soluble polymeric carrier is approved as an additive for pharmaceutical preparations, it does not particularly cause safety hazard. Accordingly, the upper limit of the use amount thereof is not particularly limited. The use amount of the water-soluble polymeric carrier should be determined within an amount range that is practically usable as a pharmaceutical product, and it is suitably about 90% by mass. The use amount of the water-soluble polymeric carrier is preferably 80% by mass.

Taking into consideration determination of the suitable content of the rapamycin or a derivative thereof used as an active ingredient and the practically usable amount of pharmaceutical product additives, the water-soluble polymeric carrier is preferably used in the solid pharmaceutical preparation at a content percentage of 1% by mass or more and 90% by mass or less. More preferably, the content percentage of the water-soluble polymeric carrier is 10% by mass or more and 80% by mass or less.

As such a water-soluble polymeric carrier, a water-soluble cellulose derivative is preferably used. The water-soluble cellulose derivative is a water-soluble polymer, in which some hydrogen atoms in the hydroxyl group of cellulose are substituted with substituents such as methyl groups, ethyl groups, propyl groups, hydroxypropyl groups, or carboxymethyl groups.

The water-soluble cellulose derivative used in the present invention is preferably a water-soluble cellulose derivative that is approved as a pharmaceutical product additive. Specific examples of such a water-soluble cellulose derivative include hydroxypropylmethyl cellulose, hydroxypropyl cellulose, methyl cellulose, ethyl cellulose, hydroxyethyl cellulose, hydroxymethylethyl cellulose, cellacefate, hydroxypropylmethyl cellulose phthalate, hypromellose acetate succinate, hypromellose phthalic ester, carboxymethyl cellulose, and the alkali metal salts thereof, such as sodium salts or potassium salts. Preferred examples include hydroxypropylmethyl cellulose, hydroxypropyl cellulose, methyl cellulose, ethyl cellulose, and hypromellose phthalic ester. Among these, hydroxypropylmethyl cellulose is most preferable.

In the solid pharmaceutical preparation of the present invention, in the case of using the above-described water-soluble cellulose derivative, the water-soluble cellulose derivative is preferably added, at a content percentage of 1% by mass or more, into the solid pharmaceutical preparation. Preferably, the water-soluble polymeric carrier may be used at a content percentage of 10% by mass or more. If the water-soluble polymeric carrier is approved as an additive for pharmaceutical preparations, it does not particularly cause safety hazard. Accordingly, the upper limit of the use amount thereof is not particularly limited. The use amount of the water-soluble cellulose derivative should be determined within an amount range that is practically usable as a pharmaceutical product, and it is suitably about 80% by mass. The use amount of the water-soluble cellulose derivative is preferably 50% by mass.

Taking into consideration determination of the suitable content of the rapamycin or a derivative thereof used as an active ingredient and the practically usable amount of pharmaceutical product additives, the water-soluble cellulose derivative is preferably used in the solid pharmaceutical preparation at a content percentage of 1% by mass or more and 80% by mass or less. More preferably, the content percentage of the water-soluble polymeric carrier is 10% by mass or more and 50% by mass or less.

In the present invention, the solution of rapamycin or a derivative thereof may comprise other additives that are commonly used in preparation of pharmaceutical product formulations so long as it does not impair the effects of the present invention. Such other additives which can be applied may comprise, for example, an excipient, a disintegrator, a binder, a lubricant, a pH adjuster, inorganic salts, and a solvent.

Examples of the excipient include sugars such as lactose, maltose, mannitol, sucrose, erythritol, sorbitol, fucose, xylitol, fructose, inositol, and starch.

Examples of the disintegrator include carmellose, crospovidone, low-substituted hydroxypropyl cellulose, sodium starch glycolate, carmellose calcium, and croscarmellose sodium.

Examples of the binder include hydroxypropyl cellulose, hypromellose, polyvinyl alcohol, and polyvinyl pyrrolidone.

Examples of the lubricant include magnesium stearate, calcium stearate, sodium stearyl fumarate, talc, and sucrose fatty acid ester.

Examples of the pH adjuster include hydrochloric acid, sulfuric acid, phosphoric acid, citric acid, tartaric acid, malic acid, mesylic acid, tosylic acid, and besylic acid. A buffer, which comprises, as a main ingredient, such an acidic additive, and further, an alkaline metal salt, an alkaline-earth metal salt, or an ammonium salt, may also be used.

Examples of the inorganic salts include calcium chloride, sodium chloride, calcium oxide, and magnesium sulfate.

In general, examples of the solvent include water, a normal saline, a 5% glucose or mannitol aqueous solution, a water-soluble organic solvent (e.g., a single solvent such as glycerol, ethanol, dimethyl sulfoxide, N-methylpyrrolidone, polyethylene glycol, Cremophor, or a mixed solvent thereof), and polyethylene glycols (e.g., polyethylene glycol 300, polyethylene glycol 400, polyethylene glycol 600, polyethylene glycol 4000, etc.).

These additives can be used without any particular limitation, as long as they have purity that is acceptable for the intended use as pharmaceutical product formulations. These additives may be used alone, or may also be used as a mixture of the additives. When such other additives are used, they are used preferably in a content of 0.01 to 10% by mass in the solid pharmaceutical preparation.

The solid pharmaceutical preparation of the present invention is produced by preparing a solid dispersion comprising rapamycin or a derivative thereof, an antioxidant, and sugars having a critical relative humidity at 25°C of 95% or more, and then mixing the solid dispersion with the above-described iron oxide. The present solid pharmaceutical preparation is preferably a solid pharmaceutical preparation, in which the solid dispersion and the iron oxide are each dispersed in the solid pharmaceutical preparation.

The solid dispersion means a granulated substance, which is prepared by mixing rapamycin or a derivative thereof with an antioxidant, uniformly, and preferably at a molecular level, and then mixing the obtained mixture with sugars having a critical relative humidity at 25°C of 95% or more, to obtain an integrated product. That is to say, upon preparation of the solid pharmaceutical preparation, it is preferable to adopt a formulation step of previously preparing a solid dispersion, and then mixing the prepared solid dispersion with the above-described iron oxide and any given additives for pharmaceutical preparations to prepare a solid pharmaceutical preparation.

The antioxidant applied to the above-described solid dispersion has the same definitions as those described above. With regard to the amount of the antioxidant applied, the antioxidant can be used, as appropriate, in an amount that does not impair the stability of rapamycin or a derivative thereof. As a preferred additive amount, the antioxidant is preferably used in an amount of 0.0001 to 10 parts by mass, with respect to 1 part by mass of the rapamycin or a derivative thereof. The additive amount of the antioxidant is more preferably 0.0005 to 1.0 parts by mass, and further preferably 0.001 to 1.0 part by mass.

Besides, the above-described antioxidant is preferably applied in a state in which it is added into a solution containing the rapamycin or a derivative thereof and then, is completely dissolved therein.

The sugars having a critical relative humidity at 25°C of 95% or more, which are applied to the above-described solid dispersion, have the same definitions as those described above. With regard to the amount of the sugars applied, the sugars may be used in an amount of 0.5 parts by mass or more, with respect to 1 part by mass of the rapamycin or a derivative thereof. The sugars may be used in an amount of preferably 1 part by mass or more, and further preferably 2 parts by mass or more. It is sufficient, if the sugars are used in an amount of 5 parts by mass or more. In the present invention, since the sugars having a critical relative humidity at 25°C of 95% or more do not particularly cause safety hazard, the upper limit of the use amount thereof is not particularly limited. The use amount of the sugars should be determined within an amount range that is practically usable as a pharmaceutical product.

Taking into consideration the ensuring of the stability of the rapamycin or a derivative thereof and the practically usable amount of pharmaceutical product additives, the sugars are preferably used in an amount of 0.5 to 100 parts by mass, with respect to 1 part by mass of the rapamycin or a derivative thereof. The amount of the sugars is preferably 0.5 to 50 parts by mass, more preferably 1 to 50 parts by mass, and further preferably 2 to 50 parts by mass.

The solid dispersion can be prepared by dissolution of, or suspension and dispersion of the rapamycin or a derivative thereof, the antioxidant, and the sugars having a critical relative humidity at 25°C of 95% or more, in a suitable solvent, and then removing the solvent from the resulting solution.

The solvent used to prepare the solid dispersion is not particularly limited, and any solvent can be used, as long as, at least, the rapamycin or a derivative thereof and the antioxidant can be dissolved therein. Examples of such a solvent include water, methanol, ethanol, 1-propanol, 2-propanol, 1-butanol, 2-butanol, 2-methyl-1-propanol, 3-methyl-1-butanol, 1-pentanol, ethylene glycol, glycerin, formic acid, acetic acid, acetone, methyl ethyl ketone, methyl isobutyl ketone, anisole, methyl acetate, ethyl acetate, ethyl formate, propyl acetate, isopropyl acetate, n-butyl acetate, isobutyl acetate, acetonitrile, diethyl ether, t-butyl methyl ether, tetrahydrofuran, 1,4-dioxane, diisopropyl ether, pentane, hexane, heptane, N,N-dimethylformamide, N,N-dimethylacetamide, N-methylpyrrolidone, 1,3-dimethyl-2-imidazolidinone, dimethyl sulfoxide, dichloromethane, and chloroform.

These solvents may be used singly, or a mixed solvent consisting of two or more types of these solvents may also be applied. The present invention is not limited to the use of the above-described solvents, but the aforementioned solvents may be used as applicable preferred solvents in the present invention.

Taking into consideration the subsequent removal of the solvent, it is preferable to use a solvent having a boiling point of 120°C or lower, which can be distilled away under mild conditions. For example, water, methanol, ethanol, 1-propanol, 2-propanol, acetonitrile, acetone, methyl ethyl ketone, methyl acetate, ethyl acetate, isopropyl acetate, isobutyl acetate, tetrahydrofuran, 1,4-dioxane, pentane, heptane, diethyl ether, t-butyl methyl ether are preferable.

In addition, the solvent used in the present invention is preferably a solvent, in which the after-mentioned sugars having a critical relative humidity at 25°C of 95% or more are insoluble or hardly-soluble. Accordingly, among the above-described preferred solvents, it is particularly preferable to use methanol, ethanol, 1-propanol, 2-propanol, acetone, isopropyl acetate, and ethyl acetate. These solvents may be used each alone, or may also be used in combination.

The amount of the solvent used is not particularly limited, as long as the rapamycin or a derivative thereof is completely dissolved therein, and thus, the use amount can be adjusted, as appropriate. It is preferable to prepare a solvent, in which the rapamycin or a derivative thereof is contained in a concentration of 1 to 1000 mg/mL or less, and preferably of 10 to 500 mg/mL.

Moreover, upon preparation of the solution, the solution may be heated, as appropriate, so that dissolution of the rapamycin or a derivative thereof may be promoted. The temperature of the solution upon preparation thereof is not particularly limited. Taking into consideration the stability of the rapamycin or a derivative thereof, the solution is preferably prepared at a temperature of 0°C to 80°C.

The above-described solid dispersion comprising the rapamycin or a derivative thereof is preferably a solid dispersion comprising a water-soluble cellulose derivative. That is to say, preferably, the water-soluble cellulose derivative is allowed to coexist with a solution or a suspended dispersion of the above-described rapamycin or a derivative thereof, antioxidant, and sugars having a critical relative humidity at 25°C of 95% or more. Thereafter, the solvent is removed from the aforementioned solution to obtain a solid dispersion. At this time, the water-soluble cellulose derivative may be dissolved in, or may be suspended and dispersed in the solution.

The water-soluble cellulose derivative that can be applied to the solid dispersion has the same definitions as those described above. Preferably, hydroxypropylmethyl cellulose, hydroxypropyl cellulose, methyl cellulose, ethyl cellulose, and hypromellose phthalic ester are used, and it is more preferable to use hydroxypropylmethyl cellulose.

With regard to the additive amount of the water-soluble cellulose derivative applied to the above-described solid dispersion, the water-soluble cellulose derivative is preferably used in an amount of 0.1 part by mass or more, with respect to 1 part by mass of the rapamycin or a derivative thereof. The water-soluble cellulose derivative may be more preferably used in an amount of 0.5 parts by mass or more. That is, the above-described water-soluble polymeric carrier can be used, as appropriate, in an amount that does not impair the stability of the rapamycin or a derivative thereof. With regard to the additive amount of the above-described stabilizer, the water-soluble polymeric carrier is preferably used in an amount of 0.1 to 50 parts by mass, with respect to 1 part by mass of the rapamycin or a derivative thereof. The additive amount of the water-soluble polymeric carrier is more preferably 0.1 to 30 parts by mass, and further preferably 0.5 to 20 parts by mass.

Besides, the above-described water-soluble polymeric carrier may be in a state in which it is added into a solution containing the rapamycin or a derivative thereof and then, is completely dissolved therein, or may also be in a state in which the water-soluble polymeric carrier is suspended in the aforementioned solution. The water-soluble polymeric carrier may be used in both embodiments.

The above-described solid dispersion may further comprise, in the solution containing the rapamycin or a derivative thereof, other additives that are commonly used to prepare pharmaceutical products, within a range that does not impair the effects of the present invention. For example, the solid dispersion may comprise a pH adjuster, inorganic salts, and the like. Specific examples of other additives used as any given components have the same definitions as those described above. In the case of using other additives, such other additives are preferably used in an amount of 0.01 to 10 parts by mass, with respect to 1 part by mass of the rapamycin or a derivative thereof.

Besides, the above-described other additives may be in a state in which they are added into a solution containing the rapamycin or a derivative thereof and are completely dissolved therein, or may also be in a state in which they are suspended in the aforementioned solution. Such other additives may be used in both embodiments.

The above-described solid dispersion can be obtained by removing the solvent from a solution or a suspended dispersion comprising the rapamycin or a derivative thereof, the antioxidant, the sugars having a critical relative humidity at 25°C of 95% or more, and other additives such as any given water-soluble cellulose derivative, according to an appropriate method. As such a method of removing the solvent, a method of transpiring the solvent by heating and/or reducing the pressure is applied. At this time, it is efficient and preferable to apply a method comprising adding dropwise or spraying the above-described solution or suspended dispersion onto a suitable carrier, and then distilling away the solvent. Otherwise, a solid mixture can also be obtained by ventilation drying, in which inert gas such as air or nitrogen is supplied into a dryer and the solvent is dried.

As such a carrier, an excipient or a polymeric carrier such as cellulose, which is commonly used as a pharmaceutical preparation, is preferably used. It is to be noted that the carrier is also a constitutional component of the above-described solid dispersion.

Examples of the excipient include sugars such as lactose, maltose, mannitol, sucrose, erythritol, sorbitol, fucose, xylitol, fructose, inositol, or starch.

Examples of the polymeric carrier include hydroxypropylmethyl cellulose, hydroxypropyl cellulose, methyl cellulose, ethyl cellulose, hydroxyethyl cellulose, hydroxymethylethyl cellulose, cellacefate, hydroxypropylmethyl cellulose phthalate, hypromellose acetate succinate, hypromellose phthalic ester, carboxymethyl cellulose, and the alkali metal salts thereof, such as sodium salts or potassium salts.

As such a carrier, it is preferable to use sugars used as an excipient, and it is more preferable to use sugars having a critical relative humidity at 25°C of 95% or more. The sugars are preferably sugar alcohol or disaccharides. For example, it is preferable that one or more types of sugars selected from the group consisting of mannitol, lactose, trehalose and maltose be used as a carrier(s), and that the above-described solution or suspended dispersion be then added dropwise thereto. These sugars may be used each alone, or may also be used in combination of two or more types.

When the above-described carrier is sugars having a critical relative humidity at 25°C of 95% or more, with regard to the additive amount of the carrier, a part of the above-described preferred additive amount may be used as a carrier. Specifically, the sugars are used in a total amount in the solid dispersion that is preferably 0.5 to 100 parts by mass, more preferably 0.5 to 50 parts by mass, further preferably 1 to 50 parts by mass, and still further preferably 2 to 50 parts by mass, with respect to 1 part by mass of the rapamycin or a derivative thereof.

The solid pharmaceutical preparation of the present invention can be prepared by mixing the above-described solid dispersion, iron oxide, and any given additives for pharmaceutical preparations with one another to prepare a pharmaceutical composition, and then molding the prepared pharmaceutical composition into a solid pharmaceutical preparation according to an appropriate method. A lubricant is preferably used as any given additive for pharmaceutical preparations because such a lubricant can achieve the appropriate mixing of the solid dispersion with the iron oxide and the subsequent molding of the mixture. Examples of a suitable lubricant include magnesium stearate, calcium stearate, sodium stearyl fumarate, talc, and sucrose fatty acid ester. The lubricant is preferably used in an amount of 0.1 to 5 parts by mass, with respect to 1 part by mass of the iron oxide.

Examples of any other given additives for pharmaceutical preparations, which may be applied herein, include an excipient, a disintegrator, a binder, a lubricant, a pH adjuster, inorganic salts, and a solvent.

The solid pharmaceutical preparation of the present invention is used as a pharmaceutical product that is applied to the treatment of disease. Since the present solid pharmaceutical preparation comprises, as an active ingredient, rapamycin or a derivative thereof, the disease, to which the present solid pharmaceutical preparation can be applied, is disease on which the active ingredient is effective. For example, the solid pharmaceutical preparation of the present invention is applied to suppress rejections occurring in the transplantation of heart, lung, complex cardiopulmonary, liver, kidney, pancreas, skin, cornea, etc. Moreover, the solid pharmaceutical preparation of the present invention can also be applied to autoimmune diseases and inflammatory diseases, such as arthritis, rheumatic disease, systemic lupus erythematosus, polychondritis, scleroderma, Wegener's granulomatosis, dermatomyositis, chronic active hepatitis, myasthenia gravis, psoriasis, Stevens-Johnson syndrome, idiopathic sprue, autoimmune inflammatory bowel disease, endocrine eye disease, Graves' disease, nodule colitis, multiple sclerosis, primary biliary hepatitis, juvenile diabetes (type I diabetes), uveitis, keratoconjunctivitis sicca, vernal keratoconjunctivitis, interstitial lung fibrosis, psoriatic arthritis, glomerulonephritis, and juvenile dermatomyositis. Furthermore, the present solid pharmaceutical preparation can also be used for malignant tumors, hyperproliferative diseases and other diseases, such as breast cancer, kidney cancer, neuroendocrine tumor, lymphoproliferative disease, B cell lymph gland cancer, tuberous sclerosis, and proliferative dermatitis. The diseases, to which the solid pharmaceutical preparation of the present invention is applied, are not limited thereto, but the aforementioned diseases are preferable as diseases, to which the present solid pharmaceutical preparation can be applied.

The applied dose of a pharmaceutical product, in which the solid pharmaceutical preparation of the present invention is used, may be naturally changed depending on the sex, age, physiological conditions, pathological conditions, etc. of a patient. For instance, the rapamycin or a derivative thereof is applied at a dose of 0.01 to 100 mg/m$^2$ (body surface area) per adult per day. The applied dose is not limited to the aforementioned dose, but it may be an applicable preferred dose.

Examples

Hereinafter, the present invention will be further described in the following examples. However, these examples are not intended to limit the scope of the present invention.

It is to be noted that, in the present test examples, the analyses were performed according to high performance liquid chromatography (HPLC) under the following measurement conditions.
Measurement column: Zorbax Eclipse XDB-C18, Rapid resolution HT, 100 mm × 4.6 mm, 1.8 μm
Detector: ultraviolet absorptiometer (measurement wavelength: 278 nm) Column temperature: 45°C
Mobile phase A: 0.1% formic acid, mobile phase B: methanol/acetonitrile = 50/50

Concentration gradients of mobile phases:

[Table 1]

| Time after injection (min) | Mobile phase A (vol%) | Mobile phase B (vol%) |
|---|---|---|
| 0 - 5 | 46 | 64 |
| 5 - 17 | 46 → 25 | 64 → 75 |
| 17 - 22 | 25 → 10 | 75 → 90 |
| 22 - 24 | 10 | 90 |
| 24 - 25 | 10 → 46 | 90 → 64 |
| 25 - 28 | 46 | 64 |

Flow rate: 1.5 mL/min
Amount injected: 10 μL

[Preparation Example 1]

3.6 g of Everolimus was dissolved in 580 g of a mixed solution of acetone and ethanol. Thereafter, 100 μL of anhydrous ethanol solution of DL-α-tocopherol (Riken E Oil 1000, manufactured by Riken Vitamin Co., Ltd.) (72.7 mg/mL), 100 μL of a hexane solution of lecithin (derived from soybeans, manufactured by JUNSEI CHEMICAL CO., LTD.) (727 mg/mL), 36 g of hypromellose (manufactured by Shin-Etsu Chemical Co., Ltd.), and 3.6 g of lactose hydrate (manufactured by DFE Pharma) were added to the above obtained solution. The obtained mixture was stirred with a stirrer to prepare a spraying solution for fluidized bed granulation.
Subsequently, 98 g of anhydrous lactose (manufactured by DFE Pharma) was added into a fluidized bed granulator (FD-MP-01, manufactured by Powrex Corp.), and it was then flown with air. Thereafter, the spraying solution for fluidized bed granulation was sprayed thereon for granulation. After completion of the spraying, the granulator was continuously operated for 50 minutes for drying, and powders were then recovered to obtain Granulated Substance 1.

[Example 1]

600 mg of Granulated Substance 1, 6.0 mg of yellow ferric oxide (manufactured by Kishi Kasei Co., LTD.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Example 1.

[Example 2]

600 mg of Granulated Substance 1, 6.0 mg of ferric oxide (manufactured by Kishi Kasei Co., LTD.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Example 2.

[Example 3]

600 mg of Granulated Substance 1, 6.0 mg of yellow ferric oxide (manufactured by Kishi Kasei Co., LTD.), 6.0 mg of ferric oxide (manufactured by Kishi Kasei Co., LTD.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Example 3.

[Comparative Example 1]

600 mg of Granulated Substance 1 and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Comparative Example 1.

[Comparative Example 2]

600 mg of Granulated Substance 1, 6.0 mg of titanium oxide (manufactured by Freund Corp.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Comparative Example 2.

[Comparative Example 3]

600 mg of Granulated Substance 1 and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Comparative Example 3.

[Comparative Example 4]

600 mg of Granulated Substance 1, 6.0 mg of synthetic calcium silicate (manufactured by Tomita Pharmaceutical Co., Ltd.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Comparative Example 4.

[Comparative Example 5]

600 mg of Granulated Substance 1, 6.0 mg of talc (manufactured by NIPPON TALC Co., Ltd.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Comparative Example 5.

[Comparative Example 6]

600 mg of Granulated Substance 1, 6.0 mg of synthetic aluminum silicate (manufactured by Kyowa Chemical Industry Co., Ltd.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Comparative Example 6.

[Comparative Example 7]

600 mg of Granulated Substance 1, 6.0 mg of magnesium aluminometasilicate (manufactured by Fuji Chemical Industries Co., Ltd.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Comparative Example 7.

[Comparative Example 8]

600 mg of Granulated Substance 1, 6.0 mg of magnesium oxide (manufactured by JUNSEI CHEMICAL CO., LTD.), and 6.0 mg of magnesium stearate (manufactured by Taihei Chemical Industrial Co., Ltd.) were weighed in a polyethylene bag, and they were then mixed by shaking. Thereafter, the obtained mixture was subjected to tablet making using a single-punch tableting machine (weight: approx. 100 mg, major diameter: 12 mm, minor diameter: approx. 5 mm, and thickness: approx. 1.5 mm, per tablet), thereby preparing a solid pharmaceutical preparation of Comparative Example 8.

[Test Example 1]

The solid pharmaceutical preparations obtained in Examples 1 to 3 and Comparative Examples 1 and 2 were each placed on a petri dish, and were then preserved at 25°C under light irradiation of 1000 Lux.
On Day 7 and Day 14 after the preservation test, the amount of everolimus remaining in each solid pharmaceutical preparation was measured by high performance liquid chromatography (HPLC), and the residual percentage of everolimus at each time point was calculated. The residual percentage was calculated according to the following equation:

$$\text{Residual percentage of everolimus (\%)} = (\text{Peak area of everolimus measured by HPLC at each time point / weighed value of powder}) / (\text{Peak area of everolimus measured by HPLC before preservation (initial) / weighed value of powder}) \times 100$$

The results are shown in Table 2.

[Table 2]

| | Additive | Residual percentage of everolimus (%) | |
|---|---|---|---|
| | | 7-day value | 14-day value |
| Example 1 | Yellow ferric oxide | 91.4 | 89.7 |
| Example 2 | Ferric oxide | 86.1 | 83.7 |
| Example 3 | Yellow ferric oxide + ferric oxide | 91.4 | 90.2 |
| Comparative Example 1 | None | 82.1 | 72.3 |
| Comparative Example 2 | Titanium oxide | 76.9 | 68.1 |

[Test Example 2]

The solid pharmaceutical preparations obtained in Comparative Examples 3 to 8 were each placed on a petri dish, and were then preserved at 25°C under light irradiation of 1000 Lux.
On Day 6 and Day 14 after the preservation test, the amount of everolimus remaining in each solid pharmaceutical preparation was measured by HPLC, and the residual percentage of everolimus at each time point was calculated.

The results are shown in Table 3.

[Table 3]

| | Additive | Residual percentage of everolimus (%) | |
|---|---|---|---|
| | | 6-day value | 13-day value |
| Comparative Example 3 | None | 85.3 | 78.1 |
| Comparative Example 4 | Synthetic calcium silicate | 86.3 | 78.5 |
| Comparative Example 5 | Talc | 84.9 | 74.9 |
| Comparative Example 6 | Synthetic aluminum silicate | 85.9 | 77.5 |
| Comparative Example 7 | Magnesium aluminometasilicate | 81.8 | 70.3 |
| Comparative Example 8 | Magnesium oxide | 81.7 | 70.6 |

It has been known that, in the case of a pharmaceutical preparation containing everolimus, the decomposition reaction of the active ingredient thereof progresses under light irradiation of 1000 Lux, and that the content of everolimus decreases. It is clearly described in the interview form of everolimus preparations that commercially available everolimus preparations (Afinitor (registered trademark) tablets and Certican (registered trademark) tablets) are unstable against light.

In Comparative Example 1, in which inorganic additives such as iron oxide were not used, the decomposition of everolimus promptly progressed under light irradiation. That is, the results show that everolimus is decomposed under light irradiation, and this is a physical property shared by compounds having a rapamycin structure.

It was demonstrated that the solid pharmaceutical preparations of Examples 1 to 3 according to the present invention maintain the content of everolimus and are able to suppress the photodecomposition of everolimus. It was demonstrated that, in particular, the use of yellow ferric oxide brings on excellent light stability, and that the combined use of yellow ferric oxide and ferric oxide brings on higher light stability. In contrast, in the case of the solid pharmaceutical preparation of Comparative Example 2, to which titanium oxide known as a light-shielding agent for pharmaceutical products was added, it was observed that the added titanium oxide did not contribute to the light stability of the active ingredient, and rather tended to promote the decomposition thereof.

Furthermore, with regard to the solid pharmaceutical preparations of Comparative Examples 4 to 8 comprising inorganic additives commonly used in pharmaceutical preparations, the light stabilizing effects of everolimus were analyzed. As a result, under the light irradiation storage condition, the content of the active ingredient was equivalent to or smaller than that of the solid pharmaceutical preparation of Comparative Example 3, in which no inorganic additives were used.

From these results, it was clarified that, in order to improve the light stability of everolimus, iron oxide-based additives specifically exhibit light stabilizing effects, and other inorganic additives are not effective or rather, promote decomposition.

Yellow ferric oxide and ferric oxide are collected in the Japanese Pharmaceutical Excipients Directory, etc., and these are additives, the safety of which has been guaranteed. Therefore, the present invention can provide a solid pharmaceutical preparation comprising, as an active ingredient, rapamycin and a derivative thereof, such as everolimus, wherein the solid pharmaceutical preparation is excellent in terms of light stability and has high safety.

**Claims**

1.  A solid pharmaceutical preparation comprising rapamycin or a derivative thereof and iron oxide, wherein the iron oxide is dispersed in the solid pharmaceutical preparation.

2.  The solid pharmaceutical preparation according to claim 1, wherein the iron oxide is ferric oxide and/or yellow ferric oxide.

3.  The solid pharmaceutical preparation according to claim 1 or 2, wherein the content percentage of the iron oxide in the solid pharmaceutical preparation is 0.01% by mass or more and 2% by mass or less.

4.  The solid pharmaceutical preparation according to any one of claims 1 to 3, which comprises sugars having a critical relative humidity at 25°C of 95% or more.

5. The solid pharmaceutical preparation according to any one of claims 1 to 4, wherein the rapamycin or a derivative thereof is dispersed in the solid pharmaceutical preparation in the form of a solid dispersion comprising an antioxidant and sugars having a critical relative humidity at 25°C of 95% or more, and the iron oxide is dispersed in the solid pharmaceutical preparation in the form of a mixture with the solid dispersion.

6. The solid pharmaceutical preparation according to claim 5, wherein the solid dispersion comprising the rapamycin or a derivative thereof comprises a water-soluble cellulose derivative.

7. The solid pharmaceutical preparation according to claim 5 or 6, wherein the solid dispersion comprising the rapamycin or a derivative thereof, the iron oxide, and a lubricant are dispersed in the solid pharmaceutical preparation.

# INTERNATIONAL SEARCH REPORT

| International application No. |
| --- |
| PCT/JP2017/019192 |

A. CLASSIFICATION OF SUBJECT MATTER
*A61K31/436*(2006.01)i, *A61K47/02*(2006.01)i, *A61K47/26*(2006.01)i, *A61K47/36* (2006.01)i, *A61P31/04*(2006.01)n, *A61P35/00*(2006.01)n

According to International Patent Classification (IPC) or to both national classification and IPC

B. FIELDS SEARCHED

Minimum documentation searched (classification system followed by classification symbols)
A61K31/436, A61K47/02, A61K47/26, A61K47/36, A61P31/04, A61P35/00

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
| --- | --- | --- |
| Y | Drugs in Japan Ethical Drugs edition of the year 2009, Jiho, Inc., 01 September 2008 (01.09.2008), page 548 | 1-7 |
| Y | JP 2013-35797 A (Kyowa Pharmaceutical Industry Co., Ltd.), 21 February 2013 (21.02.2013), claims; examples (Family: none) | 1-7 |
| Y | WO 2016/076280 A1 (Shionogi & Co., Ltd.), 19 May 2016 (19.05.2016), claims; examples & JP 16-76280 A1 | 1-7 |

| ☒ Further documents are listed in the continuation of Box C. | ☐ See patent family annex. |

\* Special categories of cited documents:
"A"  document defining the general state of the art which is not considered to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search | Date of mailing of the international search report |
| --- | --- |
| 26 June 2017 (26.06.17) | 11 July 2017 (11.07.17) |

| Name and mailing address of the ISA/ Japan Patent Office 3-4-3, Kasumigaseki, Chiyoda-ku, Tokyo 100-8915, Japan | Authorized officer |
| --- | --- |
|  | Telephone No. |

Form PCT/ISA/210 (second sheet) (January 2015)

**INTERNATIONAL SEARCH REPORT**

International application No.

PCT/JP2017/019192

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2006-306754 A  (Dainippon Sumitomo Pharma Co., Ltd.), 09 November 2006 (09.11.2006), claims; examples & TW 200819144 A claims; examples | 1-7 |
| Y | JP 2011-105694 A  (Takata Seiyaku Co., Ltd.), 02 June 2011 (02.06.2011), claims; examples 1 to 6 (Family: none) | 1-7 |
| Y | JP 2005-507897 A  (Novartis AG.), 24 March 2005 (24.03.2005), paragraphs [0078], [0107]; example 1 & US 2004/0254210 A1 paragraphs [0079], [0108]; example 1 & WO 2003/028705 A1    & EP 2193788 A1 & KR 10-2004-0037197 A   & CN 1561201 A | 4-7 |
| A | JP 2008-532953 A  (LifeCycle Pharma A/S), 21 August 2008 (21.08.2008), & US 2008/0275076 A1    & WO 2006/094507 A1 & EP 1858511 A1          & CN 101137365 A & AU 2006222409 A | 1-7 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11509223 A
- JP 2005507897 A
- JP 2002531527 A
- WO 2013022201 A
- JP 2003104887 A
- WO 94022136 A
- US 5258389 A
- WO 9409010 A
- WO 9205179 A
- US 5118677 A
- US 5100883 A
- US 5151413 A